Europäisches Patentamt

European Patent Office

Office européen des brevets

(11) Publication number: **0 042 303**
**B1**

(12) **EUROPEAN PATENT SPECIFICATION**

(45) Date of publication of patent specification: **03.04.85**

(51) Int. Cl.⁴: **A 23 K 1/18, A 23 K 1/00**

(21) Application number: **81302707.5**

(22) Date of filing: **16.06.81**

(54) **A method of improving animal coats.**

(30) Priority: **17.06.80 JP 80906/80**

(43) Date of publication of application:
**23.12.81 Bulletin 81/51**

(45) Publication of the grant of the patent:
**03.04.85 Bulletin 85/14**

(84) Designated Contracting States:
**FR GB IT**

(56) References cited:
**DE-A-1 692 503**
**DE-A-2 921 213**
**DE-C-1 016 106**
**GB-A-1 069 400**

**Patents Abstracts of Japan Vol. 5, No. 24, 13
February 1981**

(73) Proprietor: **Eisai Co., Ltd.**
**6-10, Koishikawa 4-chome Bunkyo-ku
Tokyo 112 (JP)**

(72) Inventor: **Kimura, Makoto**
**2-3-35, Ohyada
Adachi-ku Tokyo (JP)**

(74) Representative: **Ruffles, Graham Keith et al
MARKS & CLERK 57-60 Lincoln's Inn Fields
London WC2A 3LS (GB)**

Courier Press, Leamington Spa, England.

# 0 042 303

**Description**

This invention relates to a method of improving animal coats. More particularly, it relates to a method of improving the coats of domesticated livestock including sheep, goats and other animals having wool; and minks, foxes and other animals having fur.

The coats of sheep, rabbits, cows etc, are utilized in large amounts, for example as textile materials or felt materials. Enhancement of the quantity and quality of the coats is thus important for animal raisers. To produce as much of as high quality as possible is an important object, and various studies have been carried out to achieve this object by the improvement of breeds.

We have been intensively studying methods to increase production of animal coats and improve the quality, without relying on the improvement of breeds.

In other words, we have been studying for many years for the purpose of producing the greatest possible yield of the highest possible quality per animal, such as sheep or rabbit. As a result, it has now been discovered that administration of *Bacillus subtilis* var. *natto* to animals unexpectedly can produce large yields of coats of high quality.

Accordingly, this invention provides a method of improving the wool or fur of an animal which comprises feeding the animal with spores of *Bacillus subtilis* var. *natto*, the daily feed per animal being at least $10^6$ spores.

*Bacillus subtilis* var. *natto* belongs to the species *Bacillus subtilis*, which is one of the most stable bacterial species in nature.

Strains of the variety *natto* are widely available, without restrictions. Examples of commercial products thereof are, for example, Growgen (commodity name). This product is formulated into a preparation by adding pharmacopeia potato starch and contains $10^8$ or more spores of *B. subtilis* var. *natto* strain BN per gram of the formulation. In this invention, any product which contains *B. subtilis* var. *natto* in some form may be used; in particular any *B. subtilis* var. *natto* used in production of 'natto' (fermented soybean) may be employed.

The amount of *B. subtilis* var. *natto* to be used for practicing this invention can vary depending on the kind of the animal, the other components of the feed, etc, and thus cannot be stated unconditionally. However, *B. subtilis* var. *natto* is generally administered in an amount of at least $10^6$ and generally about $10^7$ to $10^{10}$ spores per day, added to animal feeds.

The base animal feed can be of conventional kind, and may be a naturally-occurring feed or a formulated feed. For preference, the base animal feed is formulated and contains at least 5%, more preferably at least 15% protein and at least 0.5%, more preferably at least 1% oil or fat. The protein can be of animal, vegetable or bacterial origin, and for instance is meat and bone, fish meal, soya beans, rape beans, peas, or 'Pruteen' (Registered Trade Mark). The oil or fat is a triglyceride, for instance tallow or other animal triglyceride or sunflower oil or other vegetable triglyceride. Usually the base feed will contain appreciable amounts of carbohydrates, including sugars and cellulosic fibres, together with lesser amounts of minerals vitamins, antibiotics or other additives. Knowing the amount of base feed typically consumed per day by the recipient animal, it is a simple matter to formulate the feed to contain a suitable dosage of the *B. subtilis* var. *natto*.

The animal for which this invention is contemplated may be any animal with a coat; specific examples include those providing textile materials, felt materials, writing or other brush materials, and other hair products, such as sheep, rabbits, camels, goats and the like livestock; those providing fur materials such as minks, weasels, foxes, raccoon dogs, and the like fur animals; pet animals such as dogs, cats, etc.

Among the above, those of the greatest value from an economic view point are sheep, rabbits, goats and minks. In the case of sheep, representative examples include wool breeds such as Rambouillet merino, Australian merino, American merino, Derian merino, Saxony merino, Schiredia merino, Hungarian merino, Hanover merino and other Merinos; wool and mutton breed such as Corriedale, Borarse, Roameldale, Corombia, Montadale, Panama, Perendale, Ideal, Karsdale, Waletenberg; and other breeds such as Chinese sheep, Mongolian sheep, Romanoff, Karakul, etc. Representative examples of rabbits are such species as Angora, Japanese white, New Zealand white, etc. Representative examples of goats are wool breeds such as Angora, Cashmere, etc. Representative examples of minks are Pastel, Sapphire, Dark species, etc.

In the case of wool, a product having longer and thinner staples and a greater crimp number is generally regarded as superior. The "crimp" is a phenomenon in which each wool staple exhibits waviness twisted right and left along its longitudinal axis. The distance between the crest of one wave and the crest of the next wave is counted as 'one', and the numerical value of such distances in a unit length is referred to as the crimp number. Wool having a greater crimp number is preferred since it improves the handfeel and touch of wool and enhances the heat retaining properties by making a space between the staples. Therefore the crimp number is an important index to determine the quality of wool as a textile material.

By administering *B. subtilis* var. *natto* to animals according to this invention, the coat yield and quality can be enhanced and thus coat performance and production is greatly improved.

In other words, by practicing this invention, as demonstrated in the example given herein below, it has now been made possible to produce hair having longer staples and a greater crimp number as well, and furthermore, it lends a very fleecy coat apparent to the naked eyes, thus presenting outstanding beauty. Therefore, hair obtained from sheep, rabbits, camels, etc, according to this invention is ideal as textile

2

## 0 042 303

materials and furthermore hair obtained from sheep, goats, minks, foxes, weasels, raccoon dogs, etc, is most suitable as hair materials. In addition, as described above, since this invention imparts a fine coat apparent to the naked eyes and very attractive in appearance, this invention is also very desirable for use for pet animals such as dogs, cats, etc. *B. subtilis* var. *natto* used in this invention is extremely economic, has no side effects and further *B. subtilis* var *natto* itself has a digestive effect, and therefore it is very suitable as an animal feed additive.

The effect of this invention is more particularly described in the following non-limiting example.

### Example

Results of the Tests on Sheep
1. Animals Tested

Ten 2-month old sheep naturally crossbred from the Corriedale and Suffolk species and each weighing approximately 25 kg were used.

2. Raising

The animals were individually raised in sheep pens each 1.7 m 1.7 m, that is 2.89 m².

3. Animal Feed Supplied

A concentrated feed for cows (a commercially-available feed for fattening cows in the later stages; commodity name: New King Beef) and hay were used. The analysis for the components of the hay and feed were as set forth in Table 1 below.

### TABLE 1

| | Component | | | | | | |
|---|---|---|---|---|---|---|---|
| Animal Feed | Water Content | Crude Protein | Crude Fat | Crude Fiber | NFE | Crude Ash | Total |
| Hay | 15.06 | 7.21 | 1.70 | 33.97 | 37.71 | 4.35 | 100.00 |
| Concentrated Animal Feed | 15.50 | 11.54 | 1.51 | 1.05 | 65.90 | 4.50 | 100.00 |

4. Method of Administration

A *B. subtilis* var. *natto* preparation (commodity name: Growgen 8) was added to the above described concentrated feed in an amount of 10 g per day per animal, corresponding to $10^9$ or more spores of *B. subtilis* var. *natto* strain BN, and was administered for 4 months to sheep in a Test Group. Sheep in a Control Group received the hay and feed without the *B. subtilis* var. *natto*.

5. Test Results

At the end of the 4 months, the staple length, staple thickness and crimp number for the wool from each sheep were measured. The respective results are given in Tables 2 to 4 below.

### TABLE 2
#### Staple length on the Neck Side (cm)

| Group | Sheep Number | | | | | Mean Value ± Standard Deviation |
|---|---|---|---|---|---|---|
| | 1 | 2 | 3 | 4 | 5 | |
| Control Group | 6.3 | 7.5 | 6.8 | 7.8 | 8.0 | 7.28 ± 0.71 |
| Test Group | 10.1 | 10.2 | 9.6 | 8.9 | 10.3 | 9.82 ± 0.58 *** |

***$P < 0.001$

As is evident from Table 2, the mean staple length for the test group was about 2.5 cm more, as compared with the control group.

## 0 042 303

### TABLE 3

Staple Thickness (average Diameter of 20 staples, microns)

| Group | Sheep Number | | | | | Mean Value ± Standard Deviation |
|---|---|---|---|---|---|---|
| | 1 | 2 | 3 | 4 | 5 | |
| Control Group | 35.4 | 32.5 | 34.5 | 32.0 | 31.8 | 33.24 ± 1.61 |
| Test Group | 28.0 | 27.2 | 30.2 | 31.5 | 26.9 | 28.76 ± 2.00** |

** $P < 0.01$

As is evident from Table 3, the mean staple thickness for the test group was about 4.5 microns less, compared with the control group.

### TABLE 4

Crimp Numbers per Inch

| Group | Sheep Number | | | | | Mean Value ± Standard Deviation |
|---|---|---|---|---|---|---|
| | 1 | 2 | 3 | 4 | 5 | |
| Control Group | 9 | 9 | 9 | 9 | 10 | 9.2 ± 0.45 |
| Test Group | 10 | 10 | 10 | 10 | 11 | 10.2 ± 0.45** |

** $P < 0.01$

As is evident from Table 4, the mean crimp number per inch for the test group was about one larger than that for the control group.

In addition, macrographic observations also revealed that the sheep of the test group had finer and more fleecy coats and better physique, compared with those of the control group.

As demonstrated by the above experimental example, the practice of this invention enables production of wool having thinner and longer staples with an increased crimp number; in addition it is apparent to the naked eye that the coats obtained are very fleecy, and thus have outstanding appeal. Therefore, this invention is of great value.

**Claims**

1. A method of improving the wool or fur of an animal which comprises feeding the animal with spores of *Bacillus subtilis* var. *natto*, the daily feed per animal being at least $10^6$ spores.

2. A method according to claim 1, wherein the daily feed per animal is $10^9$ or more spores.

3. A method according to claim 1, wherein the animal is a sheep.

4. A method according to claim 1, wherein the animal is a rabbit.

5. A method according to claim 1, wherein the animal is a mink.

**Patentansprüche**

1. Verfahren zur Verbesserung der Wolle oder des Felles eines Tieres durch Füttern des Tiers mit Sporen von *Bacillus subtilis* var. *natto*, wobei die tägliche Futtermenge mindestens $10^6$ Sporen pro Tier beträgt.

2. Verfahren nach Anspruch 1, dadurch gekennzeichnet, daß die tägliche Futtermenge $10^9$ oder mehr Sporen pro Tier beträgt.

3. Verfahren nach Anspruch 1, dadurch gekennzeichnet, daß das Tier ein Schaf ist.

4. Verfahren nach Anspruch 1, dadurch gekennzeichnet, daß das Tier ein Kaninchen ist.

5. Verfahren nach Anspruch 1, dadurch gekennzeichnet, daß das Tier ein Nerz ist.

4

## 0 042 303

**Revendications**

1. Procédé pour améliorer la qualité de la laine ou de la fourrure d'un animal, qui comprend l'administration à l'animal, comme nourriture, de spores de Bacillus subtilis var. natto, la dose quotidienne fournie par animal étant d'au moins $10^6$ spores.

2. Méthode selon la revendication 1, dans laquelle la quantité quotidienne fournie est par animal de $10^9$ spores ou davantage.

3. Méthode selon le revendication 1, dans laquelle l'animal est un mouton.

4. Méthode selon la revendication 1, dans laquelle l'animal est un lapin.

5. Méthode selon la revendication 1, dans laquelle l'animal est un vison.